# EUROPÄISCHE PATENTSCHRIFT

(11) **EP 3 381 457 B1**
(45) Veröffentlichungstag und Bekanntmachung des Hinweises auf die Patenterteilung: **14.04.2021**
(21) Anmeldenummer: 17163233.4
(22) Anmeldetag: 28.03.2017
(51) Int. Cl.: A61K 31/7008, A61K 38/01, A61K 31/728, A61K 33/00, A61P 19/00, A61K 31/198, A61K 31/375, A61K 36/899, A61K 45/06, A61P 29/00, A23L 33/10

(54) **PHYSIOLOGISCH AKTIVE ZUBEREITUNG UMFASSEND N-ACETYL-GLUKOSAMIN ZUR BEHANDLUNG VON RÜCKENSCHMERZEN**
PHYSIOLOGICALLY ACTIVE COMPOSITION
COMPOSITION PHYSIOLOGIQUE ACTIVE

(43) Veröffentlichungstag der Anmeldung: 03.10.2018
(73) Patentinhaber: Natural Products & Drugs GmbH, 9800 Spittal/Drau (AT)
(72) Erfinder: Schantl, Josef, 9871 Seeboden (AT)
(74) Vertreter: Rothkopf, Ferdinand

(56) Entgegenhaltungen:
- DE-U1-202015 101 782
- Dr. Loges: "arthroLoges protect", , 16. Juni 2016 (2016-06-16), Seiten 1-2, XP055391800, Gefunden im Internet: URL:https://www.besamex.de/images/products /pharmaVersand/beipackzettel/PB_arthroLoge s_protect.pdf [gefunden am 2017-07-18]
- Anonymous: "Document properties of arthroLoges protect", , 18. Juli 2017 (2017-07-18), Seite 1, XP055391807, Gefunden im Internet: URL:https://www.besamex.de/images/products /pharmaVersand/beipackzettel/PB_arthroLoge s_protect.pdf [gefunden am 2017-07-18]
- DAIKI KUBOMURA ET AL: "Effect of N-acetylglucosamine administration on cartilage metabolism and safety in healthy subjects without symptoms of arthritis: A case report", EXPERIMENTAL AND THERAPEUTIC MEDICINE, 21. Februar 2017 (2017-02-21), XP055391881, GR ISSN: 1792-0981, DOI: 10.3892/etm.2017.4140

## Beschreibung

### Hintergrund der Erfindung

Die Erfindung betrifft eine physiologisch aktive Zusammensetzung zur Anwendung in einem Verfahren zur Prophylaxe und/oder Behandlung von Rückenschmerzen. Ferner betrifft die Erfindung eine physiologisch aktive Zusammensetzung, die mindestens zwei Komponenten umfasst, von denen eine erste Komponente Kieselsäure ist.

Immer mehr Menschen sind in immer jüngeren Jahren von Rückenschmerzen in Ruhe und/oder unter Belastung betroffen. Ursachen für solche Rückenschmerzen sind zusätzlich zu altersbedingten Abnutzungserscheinungen zu wenig Bewegung, zu viele Belastungen und/oder falsche Belastungen. So kann stundenlanges Sitzen bei Büroarbeit zu massiven Rückenschmerzen führen.

Solche Rückenschmerzen beruhen in der Regel auf degenerativen bzw. verschleißbedingten Wirbelsäulenveränderungen, die zu Wirbelsäulenbeschwerden führen. Eine Wirbelsäule umfasst mehrere Wirbel aus je einem Wirbelkörper und einem Wirbelbogen sowie jeweils eine zwischen zwei Wirbeln sich befindende Bandscheibe. Mit dazugehörigen Wirbelsäulenbändern sind die Wirbel und Bandscheiben miteinander verbunden und stabilisiert.

An den Bandscheiben beginnt ein Verschleiß schon ab etwa dem 20. Lebensjahr eines Menschen mit Abschluss des Wachstums. Dabei verlieren die Bandscheiben nach und nach ihre Dämpfungsfunktion zwischen den Wirbeln. Eine zunehmende Abnützung und Veränderung der Wirbel sowie Muskelverhärtungen durch eine damit einhergehende veränderte Wirbelsäulenstatik sind die Folge. Auch besteht die Gefahr eines Bandscheiben-Vorfalls. Damit verbunden sind zumindest teilweise andauernde Schmerzen in Ruhe und/oder unter Belastung. Der Verschleiß der Bandscheiben ist für etwa 70 Prozent aller Rückenbeschwerden die Ursache.

Auf der Suche nach Linderung greifen betroffene Personen oft zu frei verkäuflichen oder rezeptpflichtigen Medikamenten, unterziehen sich langwierigen physiotherapeutischen Behandlungen oder sogar chirurgischen Eingriffen.

Aus DE 20 2015 101782 U1 ist eine Zusammensetzung bekannt, die zur Erhaltung und/oder Verbesserung des Zustandes der Knochen in Gelenken und/oder des Knorpels und/oder der Bänder und/oder der Sehnen und/oder der Muskeln und Faszien des menschlichen Körpers dienen soll. Die Zusammensetzung umfasst Collagenhydrolysat, Vitamin C und Kieselsäure.

In DAIKI KUBOMURAETAL: "Effect of N-acetylglucosamine administration on cartilage metabolism and safety in healthy subjects without symptoms of arthritis: A case report", EXPERIMENTAL AND THERAPEUTIC MEDICINE, 21. Februar 2017 (2017-02-21), GR, ISSN: 1792-0981, DOI: 10.3892/etm.2017.4140 sind Ergebnisse einer Studie beschrieben, die sich auf Arthrose von Knien bezieht. Die Ergebnisse weisen darauf hin, dass oral verabreichtes N-Acetyl-Glukosamin den Metabolismus von Gelenkknorpel verbessert.

### Zugrundeliegende Aufgabe

Der Erfindung liegt die Aufgabe zugrunde, eine Zusammensetzung bereitzustellen, die wirkungsvoll der Prophylaxe und/oder Behandlung von Rückenschmerzen dient, die auf degenerativen Veränderungen der Bandscheiben beruhen. Damit sollen degenerative Wirbelsäulenbeschwerden verhindert oder gelindert werden.

### Erfindungsgemäße Lösung

Diese Aufgabe ist erfindungsgemäß mit einer N-Acetyl-Glukosamin umfassenden physiologisch aktiven Zusammensetzung zur Anwendung in einem Verfahren zur Prophylaxe und/oder Behandlung von Rückenschmerzen gelöst.

N-Acetyl-Glukosamin (NAG) bzw. N-Acetyl-D-Glukosamin ist das Amid von Glukosamin und Ethansäure bzw. Essigsäure. Andere Namen für N-Acetyl-Glukosamin sind 2-Acetamido-2-desoxy-D-glucopyranose bzw. 2-Acetamido-2-deoxy-ß-glucopyranose bzw. 2-(Acetylamino)-2-deoxy-D-glucose. Als ein Derivat von N-Acetyl-Glukosamin ist ein Derivat zu verstehen, bei dem N-Acetyl-Glukosamin als Grundstruktur erhalten ist. Insbesondere ist an Stelle eines H-Atoms des N-Acetyl-Glukosamins ein anderes Atom oder eine andere Atomgruppe vorgesehen.

Mittels der erfindungsgemäßen N-Acetyl-Glukosamin umfassenden Zusammensetzung kann ein überraschend guter Effekt bei der Prophylaxe und/oder Behandlung von Rückenschmerzen erreicht werden.

N-Acetyl-Glukosamin ist aufgrund der Bindung seines Stickstoffs im Ethansäureamid besonders stabil gegenüber einer Reaktion mit anderen Substanzen. Zudem wurde erfindungsgemäß festgestellt, dass N-Acetyl-Glukosamin im Körper besonders schnell und in hoher Menge bioverfügbar ist.

Ein Vergleich des N-Acetyl-Glukosamins mit Glukosamin zeigt, dass Glukosamin in freier Form nicht vorkommt. Glukosamin ist ein herkömmliches Nahrungsergänzungsmittel und Arzneimittel, das zur Förderung der Gelenkgesundheit und bei Arthrose des Kniegelenks verwendet wird. Dort liegt das Glukosamin immer als Glukosaminsulfat oder Glukosaminhydrochlorid vor, das zusätzlich durch den Zusatz von Kaliumchlorid oder Natriumchlorid stabilisiert ist. Ansonsten würde Glukosamin aufgrund von freien Valenzen seines Stickstoffs mit sich selbst und anderen Substanzen reagieren.

Ferner wurde insbesondere im Vergleich zu peroral eingesetztem Glukosaminsulfat eine schnellere und höhere Bioverfügbarkeit des N-Acetyl-Glukosamins festgestellt. Die Bioverfügbarkeit des N-Acetyl-Glukosamins ist mehr als dreimal so hoch wie die Bioverfügbarkeit von Glukosaminsulfat, gemessen daran, wie viel der jeweiligen Substanz tatsächlich im Gewebe nach einer oralen Aufnahme verfügbar ist.

Glukosamin kann im Körper zu N-Acetyl-Glukosamin umgewandelt werden, jedoch kostet eine solche Umwandlung Zeit. Zudem geht ein Teil von oral aufgenommenem Glukosamin verloren, das vom Körper als Energiequelle verbrannt oder direkt wieder ausgeschieden wird.

Mit der N-Acetyl-Glukosamin umfassenden erfindungsgemäßen Zusammensetzung kann dem menschlichen Körper ein direkter Vorläufer von Hyaluronsäure und von anderen Glykosaminoglykanen (GAG) bzw. Mukopolysacchariden bereitgestellt werden. Damit kann eine Produktion der Hyaluronsäure und anderer Glykosaminoglykane im Körper effektiv gesteigert werden. Hyaluronsäure weist als Glykosaminoglykan eine makromolekulare Kette aus einer sich wiederholenden Disaccharid-Einheit auf, die aus D-Glucuronsäure und N-Acetyl-D-Glukosamin gebildet ist. Hyaluronsäure kommt in verschiedenen Gewebearten vor und erfüllt viele Funktionen. Vor allem das Speichern von Wasser in den unterschiedlichen Geweben ist eine herausragende Funktion der Hyaluronsäure.

Besonders positive Auswirkungen einer solchen Förderung der Hyaluronsäureproduktion im Körper bei Zufuhr des N-Acetyl-Glukosamins wurden auf die Bandscheiben festgestellt.

Die Bandscheibe ist eine Verbindung zwischen zwei Wirbeln aus Faserknorpelgewebe bzw. Faserknorpel. Eine solche Verbindung ist eine knorplige Knochenverbindung bzw. Symphyse. Die Symphyse ist ein unechtes Gelenk und unterscheidet sich grundsätzlich von einer faserknorpligen Zwischengelenkscheibe in einem echten Gelenk, wie in einem Kniegelenk, Schultergelenk, Hüftgelenk, usw. Bei dem echten Gelenk befindet sich zwischen zwei Knochenenden ein Gelenkspalt und die Knochenenden sind von einer Knorpelschicht überzogen. Zwischen den Knorpelschichten befindet sich die Zwischengelenkscheibe, die aus Faserknorpel und einem straffen, parallelfaserigen Bindegewebe aufgebaut ist.

Demgegenüber umfasst die Bandscheibe einen äußeren Faserring und einen inneren Gallertkern. Der Faserring weist außen konzentrische Schichten aus kollagenen Bindegewebsfasern auf, die nach innen in Faserknorpel übergehen. Die Bindegewebsfasern heften an den Wirbeln an und verbinden so die Wirbel untereinander. Im Inneren des Faserrings befindet sich der Gallertkern, der ein gallertartiges Gewebe ist. Das Gewebe enthält 80 bis 85 Prozent Wasser und eine Matrix aus Hyaluronsäure und Proteoglykanen, deren Seitenketten Glykosaminoglykane sind. Damit kann das Gewebe verhältnismäßig große Mengen an Wasser reversibel binden und als eine Art Wasserkissen stoßdämpfend wirken.

Im Laufe eines Lebens verschleißen die Bandscheiben zusehends. Dabei verliert der Gallertkern seine Matrix aus Hyaluronsäure und Proteoglykanen. Der Gallertkern trocknet zunehmend aus, wodurch sich sein Volumen verringert und die Wirbelkörper sich aneinander annähern. Zudem geht dadurch die Dämpfungsfunktion der Bandscheiben verloren. Die Wirbelkörper werden übermäßig belastet und abgenützt, was zu Rückenschmerzen führt.

Bei Zufuhr von N-Acetyl-Glukosamin konnte beobachtet werden, dass Rückenschmerzen, die auf degenerativen Veränderungen der Bandscheiben beruhen, zuverlässig nachlassen und behoben werden können. N-Acetyl-Glukosamin steigert die Bildung von Hyaluronsäure und Proteoglykanen im Gallertkern effektiv und verbessert die Wasserspeicherfunktion des Gallertkerns. Zudem wirkt N-Acetyl-Glukosamin dort entzündungshemmend und schmerzlindernd. Damit können die Bandscheiben mittels N-Acetyl-Glukosamin besonders effektiv erhalten und/oder aufgebaut werden. Ein Abstand zwischen den Wirbelkörpern kann wieder vergrößert werden. Solche Ursachen von Rückenschmerzen können entsprechend effektiv behandelt werden. Im Idealfall kann sogar den Rückenschmerzen vorgebeugt werden.

Erfindungsgemäß vorteilhaft umfasst die Zusammensetzung zusätzlich Kieselsäure. Der Begriff Kieselsäure umfasst im Sinne vorliegender Erfindung Kieselsäure mit der allgemeinen Summenformel H₂ₙ₊₂SiₙO₃ₙ₊₁, amorphe Kieselsäure, amorphes bzw. nichtkristallines Siliziumdioxid, natürliche bzw. organische Kieselsäure und natürliches Siliziumdioxid. Kieselsäure ist also insgesamt ein Lieferant für Silizium, das ein für den Menschen essentielles Spurenelement ist. Silizium wird im Körper für eine Produktion von Keratin in Haaren und Nägeln, für eine Wundheilung und für eine Regeneration von Bindegewebe benötigt. Dazu wirkt Silizium stimulierend auf eine Synthese von Glykosaminoglykanen und von Collagen, einem faserförmigen Strukturprotein des Bindegewebes. Damit trägt Silizium zur Elastizität des Bindegewebes bei und sorgt für dessen Stabilität.

In der erfindungsgemäß vorteilhaften Kombination von Kieselsäure mit N-Acetyl-Glukosamin ist eine besonders wirksame Zusammensetzung zur Anwendung in einem Verfahren zur Prophylaxe und/oder Behandlung von Rückenschmerzen bereitgestellt. Die Kieselsäure verstärkt die Wirkung von N-Acetyl-Glukosamin dahingehend, dass eine Vernetzung der Glykosaminoglykane, von Collagen und Elastin, einem weiteren faserförmigen Strukturprotein gefördert wird. Ein Zusammenwirken der derart vernetzten Körperstrukturen dämpft auftretende Stöße besonders stark. Auf die Bandscheiben und Wirbel auftretende Kräfte können damit besonders schnell und umfassend in die Wirbelsäulenbänder und weiteren elastischen Strukturen der Wirbelsäule abgeleitet werden.

Zudem bewirkt die Kieselsäure, dass der Faserring der Bandscheibe für seine Funktion elastisch bleibt. Der Faserring umfasst Bindegewebe, das mittels des Siliziums der Kieselsäure stabilisiert werden kann. Zudem hat N-Acetyl-Glukosamin positive Auswirkungen auf die Wasserspeicherfunktion des Bindegewebes mittels Stimulation einer Hyaluronsäurebildung im Bindegewebe. Damit kann verhindert werden, dass der Faserring austrocknet und spröde wird. Ein Riss des Faserrings würde andernfalls zu einem Bandscheibenvorfall führen. Bei einem Bandscheibenvorfall tritt der Gallertkern in einen durch übereinanderliegende Wirbel gebildeten Wirbelkanal aus. In dem Wirbelkanal befindet sich das Rückenmark, auf das der Gallertkern dann drückt und Schmerzen verursacht.

Erfindungsgemäß ist die Kieselsäure besonders vorteilhaft als Bestandteil eines Bambus-Extrakts vorgesehen. In dem Bambus-Extrakt ist natürliches Siliziumdioxid bzw. natürliche Kieselsäure reichhaltig vorhanden.

Das Bambus-Extrakt ist in herkömmlicher Weise gewonnen aus Bambus, insbesondere aus einer bestimmten Spezies des Bambus, nämlich aus Bambusa arundinacea und Bambusa vulgaris. Dazu ist eine Aussonderung aus Hohlräumen des Bambus verwendet, der sogenannte Bambuskampfer bzw. Tabashir. Tabashir ist eine siliziumreiche Substanz, die im Wesentlichen amorphe Kieselsäure beinhaltet und als Grundlage für das Bambus-Extrakt dient. Das Bambus-Extrakt umfasst damit im Wesentlichen natürliches Siliziumdioxid.

Erfindungsgemäß vorteilhaft weist das Bambus-Extrakt natürliches Siliziumdioxid mit einem Massenanteil bzw. Gewichtsanteil von 60 bis 90 Massenprozent bzw. Gewichtsprozent, bevorzugt von 70 bis 80 Gewichtsprozent und besonders bevorzugt von 75 Gewichtsprozent bezogen auf das Bambus-Extrakt auf. Damit ist ein ausreichend hoher und entsprechend gut wirksamer Anteil an Siliziumdioxid im Bambus-Extrakt enthalten.

Ferner weist das Bambus-Extrakt erfindungsgemäß vorteilhaft bezogen auf N-Acetyl-Glukosamin ein Massenverhältnis von 0,5 bis 0,9, bevorzugt von 0,6 bis 0,8 und besonders bevorzugt von 0,7 auf. Mit dem derartigen Massenverhältnis konnte eine besonders gute Wechselwirkung insbesondere bei der Behandlung von Rückenschmerzen erzielt werden.

Weiterhin umfasst die erfindungsgemäße Zusammensetzung in vorteilhafter Weise zusätzlich Collagen. Eine Zufuhr von Collagen wirkt sich positiv auf die Gesundheit von Gelenken aus und kann Osteoporose vorbeugen. Collagen ist ein faserförmiges Strukturprotein des Bindegewebes und ist das am meisten verbreitete Protein im menschlichen Körper. Als strukturgebendes Protein ist Collagen der wichtigste Aufbaustoff der Wirbelkörper und Wirbelbögen sowie aller Knorpel der Bandscheiben. Collagen ist damit wesentlich am Aufbau der Wirbelsäule beteiligt. Ferner stärkt Collagen Sehnen und Stützmuskulatur sowie die Wirbelsäulenbänder bzw. Bandverbindungen der Wirbelsäule. Die Zufuhr von Collagen in Kombination mit N-Acetyl-Glukosamin und/oder Kieselsäure verstärkt deren positive Wirkung auf die Wirbelsäulengesundheit in synergistischer Weise.

Erfindungsgemäß besonders vorteilhaft ist Collagen als Bestandteil bzw. Komponente eines Collagenhydrolysats vorgesehen. Das Collagenhydrolysat umfasst hydrolisiertes Collagen. Dabei ist Collagen enzymatisch oder chemisch aufgeschlossen und kann so vom Körper leichter verdaut und aufgenommen werden. Die positive Wirkung von Collagen kann besser ausgenutzt und beschleunigt werden. Zudem wird in den knorpelbildenden Zellen mittels Collagenhydrolysat eine Bildung von Collagen Typ II stimuliert, das als Strukturprotein von hyalinem und elastischem Knorpel fungiert.

Bevorzugt weist das Collagenhydrolysat Collagen in einem Massenanteil von 50 bis 80 Gewichtsprozent und besonders bevorzugt von 60 bis 70 Gewichtsprozent auf. Damit liegt ein hoher und entsprechend wirksamer Anteil an Collagen im Collagenhydrolysat vor.

Zum Verstärken der beschriebenen positiven Wirkung umfasst das Collagenhydrolysat bevorzugt zusätzlich Glykosaminoglykane mit einem Massenanteil zwischen 20 bis 40 Gewichtsprozent, besonders bevorzugt zwischen 25 bis 35 Gewichtsprozent.

Weiterhin umfasst die Zusammensetzung erfindungsgemäß zusätzlich Hyaluronsäure. Die herausragende Fähigkeit von Hyaluronsäure ist, besonders große Mengen an Wasser zu binden. Damit ist Hyaluronsäure entscheidend für die Wasserspeicherung im Gallertkern der Bandscheiben und für deren Funktion als Stoßdämpfer. Es wurde erfindungsgemäß beobachtet, dass die stoßdämpfende Funktion entscheidend erhalten oder verstärkt wird, wenn Hyaluronsäure in Kombination mit N-Acetyl-Glukosamin als Baustein der Hyaluronsäure zugeführt wird. Direkt zugeführte Hyaluronsäure ist schnell verfügbar und stimuliert überraschend stark zusätzlich den Aufbau von körpereigener Hyaluronsäure mittels zugeführtem N-Acetyl-Glukosamin.

Erfindungsgemäß ist Hyaluronsäure als Bestandteil eines Collagenhydrolysats vorgesehen. Bevorzugt umfasst das Collagenhydrolysat Hyaluronsäure mit einen Massenanteil zwischen 5 und 15 Gewichtsprozent und besonders bevorzugt zwischen 8 und 12 Gewichtsprozent. Ein solcher Massenanteil an Hyaluronsäure hat sich als besonders wirkungsvoll herausgestellt. Zudem ist Hyaluronsäure im Collagenhydrolysat derart eingebunden, dass bei einer oralen Aufnahme eine möglichst große Menge an Hyaluronsäure durch den Magen gelangt. Im Magen vorhandene Magensäure würde Hyaluronsäure ansonsten größtenteils zersetzen. Damit dient das Collagenhydrolysat sozusagen als ein Transportkoffer, mit dem derart säureempfindliche Hyaluronsäure durch den Magen geschleust wird. Erst im weitgehend neutralen Milieu des Zwölffingerdarms wird das Collagenhydrolysat enzymatisch gespalten. Dadurch wird Hyaluronsäure aus dem Collagenhydrolysat freigesetzt und gelangt dann vom Dünndarm in die Blutbahn und von dort in die zwischenzelluläre Gewebsflüssigkeit des ganzen Körpers.

Ferner weist das Collagenhydrolysat in erfindungsgemäß vorteilhafter Weise bezogen auf N-Acetyl-Glukosamin ein Massenverhältnis von 2,0 bis 4,0, bevorzugt von 2,5 bis 3,5 und besonders bevorzugt von 3,0 auf. Mit dem derartigen Massenverhältnis konnte eine besonders gute Wechselwirkung insbesondere bei der Behandlung von Rückenschmerzen erzielt werden.

Weiterhin umfasst die erfindungsgemäße Zusammensetzung vorzugsweise zusätzlich Lysin, insbesondere natürlich vorkommendes L-Lysin. L-Lysin ist eine essentielle Aminosäure für den Menschen. Das bedeutet, dass L-Lysin nicht vom Körper selbst gebildet wird, sondern zugeführt werden muss.

L-Lysin regt eine Bildung und eine Aktivität von knochenbildenden Zellen an. Zudem dient L-Lysin einem Wachstum der Muskeln, unterstützt eine Ausschüttung von Wachstumshormonen und stimuliert das Immunsystem. Ferner trägt L-Lysin zu einer Regelung einer Stickstoffbilanz in den Muskeln bei und fördert eine Calciumspeicherung in den Knochen, was einer Entstehung von Osteoporose verlangsamend entgegenwirkt. Mit der Zufuhr von L-Lysin zusätzlich zu N-Acetyl-Glukosamin wird eine positive Wirkung auf den gesamten Organismus erreicht, was die positive Wirkung von N-Acetyl-Glukosamin insbesondere gegen Wirbelsäulenbeschwerden zusätzlich verstärkt.

Eine besonders starke Wirkung wird erreicht bei einem erfindungsgemäß vorteilhaften Massenverhältnis von Lysin bezogen auf N-Acetyl-Glukosamin von 0,18 bis 0,38, bevorzugt von 0,23 bis 0,33, besonders bevorzugt von 0,28.

Ferner umfasst die Zusammensetzung erfindungsgemäß vorzugsweise Vitamin C. Vitamin C umfasst als Sammelbegriff L-(+)-Ascorbinsäure und ihre Derivate mit gleicher Wirkung wie L-(+)-Ascorbinsäure. Vitamin C ist ein Coenzym für das Enzym Prolyl-4-Hydroxylase, welches zur Biosynthese von Collagen benötigt wird. Zudem unterstützt Vitamin C in Kombination mit L-Lysin im Collagen eine kovalente Quervernetzung benachbarter Moleküle. Damit wird Collagen mittels Vitamin C zusätzlich stabilisiert, was stärkende Auswirkungen auf das gesamte Bindegewebe und damit auf verbindende Strukturen der Wirbelsäule in Verbindung mit den Bandscheiben hat.

Eine maßgeblich verstärkender Effekt wird erreicht bei einem erfindungsgemäß vorteilhaften Massenverhältnis von L-(+)-Ascorbinsäure bezogen auf N-Acetyl-Glukosamin von 0,20 bis 0,40, bevorzugt 0,25 bis 0,35 und besonders bevorzugt 0,30.

Bevorzugt kann die Zusammensetzung besonders wirksam in Verbindung mit entzündungshemmendenden und schmerzstillenden Arzneimitteln aufgenommen werden, die beispielsweise als Wirkstoff Diclofenac bzw. 2-{2-[(2,6-Dichlorphenyl)amino]phenyl}essigsäure) enthalten.

Besonders bevorzugt kann zusätzlich eine physiotherapeutische Behandlung insbesondere hinsichtlich einer Linderung von Rückenschmerzen durchgeführt werden. Dabei konnte festgestellt werden, dass die erfindungsgemäße Zusammensetzung einen beschleunigenden Effekt auf Erfolge der physiotherapeutischen Behandlung hat.

Ferner ist die Erfindung gerichtet auf eine physiologisch aktive Zusammensetzung, die mindestens zwei Komponenten umfasst, von denen eine erste Komponente Kieselsäure ist und eine zweite Komponente N-Acetyl-Glukosamin Der Begriff Kieselsäure umfasst vorstehend beschriebene Formen als Lieferanten für Silizium als essentielles Spurenelement.

Eine solche erfindungsgemäße Zusammensetzung hat nicht nur die bereits beschriebenen positiven Wirkungen für eine Prophylaxe und/oder Behandlung von Rückenschmerzen. Es hat sich gezeigt, dass die erfindungsgemäße Zusammensetzung in einer Kombination der beiden Komponenten Kieselsäure und N-Acetyl-Glukosamin weitere sich gegenseitig verstärkende positive Wirkungen auf den menschlichen Organismus hat. Silizium ist essentiell für die Knochenentwicklung, für die Festigkeit und Elastizität des Knorpels und anderer Bindegewebe. Silizium stärkt Haut, Haare und Nägel. Insbesondere werden solche positiven Auswirkungen mittels N-Acetyl-Glukosamin verstärkt. Die Zufuhr von N-Acetyl-Glukosamin fördert die Synthese von Hyaluronsäure als Feuchtigkeitsspeicher der Haut, des Bindegewebes und des Knorpels. Zudem ist N-Acetyl-Glukosamin ein Baustein für eine Synthese von Glykosaminoglykanen in den Bindegeweben und ein Aktivator für die derartige Synthese. Eine Vernetzung der Glykosaminoglykane wird mittels Silizium überraschend stark gefördert. Damit wirkt die erfindungsgemäße Zusammensetzung synergistisch verstärkt und erhöhend auf die Stabilität sowie Elastizität aller Arten von Bindegewebe und insbesondere auch auf Haut, Haare und Nägel. Deren Spannkraft und Dicke konnten besonders gut gestärkt werden, sodass auch einer Faltenbildung der Haut entgegengewirkt werden konnte.

Zudem stimuliert Silizium ein gesundes Haarwachstum und glänzendes Haar. Silizium trägt zu einem Härten von Zahnschmelz bei, wodurch Zähne vor Karies geschützt werden können. Zudem verhindert Silizium Zahnfleischbluten und Zahnfleischrückgang. Ferner ist Silizium wichtig für einen Mineralisationsprozess in Knochen. Silizium gehört in ionisierter Form zu den wichtigsten Ionen in Knochen bildenden Zellen und fördert die Calciumabsorption aus der Nahrung. Damit kann Silizium einen entscheidenden Beitrag gegen eine Entwicklung von Osteoporose leisten. Weiterhin verbessert Silizium die Flexibilität von Blutgefäßen und kann gegen Arterienerkrankungen wirken. Darüber hinaus hat Silizium positive Auswirkungen auf die Gesundheit von Lunge und Darm. Allgemein erniedrigt Silizium Schwindel, Kopfschmerzen, Tinnitus und Schlafstörungen und stimuliert das Immunsystem. Demgemäß können viele positive Nebenwirkungen auf den gesamten Organismus mit der erfindungsgemäßen Zusammensetzung erzielt werden, bei der Kieselsäure und N-Acetyl-Glukosamin kombiniert sind.

Erfindungsgemäß vorzugsweise ist Kieselsäure als Komponente eines Bambus-Extrakts vorgesehen. Die bereits beschriebenen Vorzüge des Bambus-Extrakts gelten entsprechend.

Für eine besonders gute Wechselwirkung weist das Bambus-Extrakt natürliches Siliziumdioxid vorteilhaft mit dem bereits beschriebenen Massenanteil von 60 bis 90 Massenprozent bzw. Gewichtsprozent, bevorzugt von 70 bis 80 Gewichtsprozent und besonders bevorzugt von 75 Gewichtsprozent bezogen auf das Bambus-Extrakt auf. Zusätzlich können damit zugleich andere für Körperfunktionen wertvolle Substanzen natürlichen Ursprungs im Bambus-Extrakt enthalten sein. Solche Substanzen sind Eisen als essentielles Spurenelement, Calcium als Mineralstoff für Knochen und Zähne sowie Cholin und Betain. Cholin ist ein lebensnotwendiger Mikronährstoff, der im Stoffwechsel des Menschen zu Acetylcholin umgewandelt wird, das als Neurotransmitter dient. Betain kann prophylaktisch gegen Arteriosklerose wirken.

Zudem ist Tabashir als Bambus-Extrakt in der tibetanischen Medizin bekannt für seine stimulierenden, adstringierenden, fiebersenkenden, stärkenden, krampflösenden und aphrodisierenden Eigenschaften.

Ferner liegt das Bambus-Extrakt bevorzugt im bereits beschriebenen Massenverhältnis bezogen auf N-Acetyl-Glukosamin vor, womit eine besonders gute Wechselwirkung erreichbar ist.

Weiterhin ist in erfindungsgemäß vorteilhafter Weise eine weitere Komponente Collagen, das bevorzugt als Bestandteil eines Collagenhydrolysats vorgesehen ist. Insbesondere wirkt Collagen zusätzlich zu den bereits genannten Vorteilen schmerzlindernd und beweglichkeitsfördernd bei Arthrose und rheumatischer Arthritis. Die derartige Wirkung wird mittels einer entzündungshemmenden Wirkung von N-Acetyl-Glukosamin auf die Gelenke in synergistischer Weise verstärkt.

Außerdem ist erfindungsgemäß eine weitere Komponente Hyaluronsäure. Hyaluronsäure hat die bereits beschriebene Fähigkeit, besonders große Mengen an Wasser zu binden. Zudem ist Hyaluronsäure ein wichtiger Baustein des Bindegewebes sowie Hauptbestandteil der Gelenkflüssigkeit und wirkt dort als Schmiermittel bei Gelenkbewegungen. Ferner hat Hyaluronsäure zusätzlich zu den beschriebenen Vorteilen eine entzündungshemmende Wirkung im Knorpelgewebe. Weiterhin fördert Hyaluronsäure eine Regeneration von Haut und Schleimhäuten. Damit kann in Kombination mit N-Acetyl-Glukosamin und Kieselsäure die positive Wirkung auf die Elastizität von Knorpel und Haut deutlich verstärkt werden.

Hyaluronsäure liegt als Bestandteil eines Collagenhydrolysats bevorzugt in dem bereits genannten Massenanteil vor.

Das Collagenhydrolysat wurde mit seinen Vorteilen bereits beschrieben, was entsprechend auch für die erfindungsgemäße mindestens zwei Komponenten umfassende Zusammensetzung gilt. Bevorzugt weist das Collagenhydrolysat bezogen auf N-Acetyl-Glukosamin das bereits genannte Massenverhältnis auf. Eine entsprechend gute Wechselwirkung kann damit erreicht werden.

Vorzugsweise ist eine weitere Komponente erfindungsgemäß Lysin, insbesondere L-Lysin. Die bereits beschriebenen Vorteile einer zusätzlichen Kombination von L-Lysin gelten entsprechend, ebenso das bevorzugte Massenverhältnis zu N-Acetyl-Glukosamin.

Zudem ist vorteilhaft eine weitere Komponente der erfindungsgemäßen Zusammensetzung Vitamin C in bevorzugt dem gleichen bereits genannten Massenverhältnis zu N-Acetyl-Glukosamin. Zusätzlich zu den beschriebenen Vorteilen wirkt Vitamin C als Antioxidationsmittel und ist notwendig für Bildung und Reparatur der Bindegewebsstrukturen von Haut und Knorpel. Die derartigen Wirkungen verstärken eine antioxidative und eine einen Abbau von Bindegewebe hemmende Wirkung von N-Acetyl-Glukosamin in synergistischer Weise.

Erfindungsgemäß vorteilhaft sind die Zusammensetzung zur Anwendung in einem Verfahren zur Prophylaxe und/oder Behandlung von Rückenschmerzen und die mindestens zwei Komponenten umfassende Zusammensetzung als orale Verabreichungsform gestaltet. Eine orale Verabreichungsform ist leicht handhabbar und wird von einem Benutzer ohne Probleme akzeptiert. Zudem kann der Benutzer einfach selbst die Zusammensetzung einnehmen. Dabei handelt es sich bevorzugt um eine feste Verabreichungsform, insbesondere in Form von Kapseln, Tabletten, Dragees, Granulaten und Pulvern. Auch eine Zugabe der Zusammensetzung zu funktionellen Lebensmitteln, wie einem Riegel für gesunde Ernährung, ist möglich. Alternativ oder zusätzlich ist auch eine flüssige Verabreichungsform, insbesondere in Form von Tropfen, stabilen Lösungen und/oder Säften möglich. N-Acetyl-Glukosamin zeichnet sich durch eine gute Wasserlöslichkeit sowie einen süßlichen Geschmack aus und ist über einen weiten pH-Bereich stabil in Lösung.

Bei Einnahme einer oralen Verabreichungsform, insbesondere in Form einer Kapsel, gelangt die erfindungsgemäße Zusammensetzung durch die Speiseröhre in den Magen. Dort werden deren Inhaltsstoffe freigesetzt, insbesondere durch Auflösen einer Kapselhülle der Kapsel. Dabei bleibt Hyaluronsäure zunächst im Collagenhydrolysat gebunden und wird dadurch gegen eine Zersetzung mittels Magensäure geschützt. Ferner bleibt Kieselsäure im Bambus-Extrakt gebunden. Erst im Zwölffingerdarm werden das Bambus-Extrakt und das Collagenhydrolysat enzymatisch mittels Amylasen und Peptidasen aus der Bauchspeicheldrüse gespalten. Damit gibt das Collagenhydrolysat, insbesondere Collagen, Hyaluronsäure frei und beide Komponenten werden in vom Körper resorbierbare Teile gespalten. Zudem gibt das Bambus-Extrakt Kieselsäure frei. Dann werden die derart aufgespaltenen Inhaltsstoffe durch die Darmwand in die Blutbahn aufgenommen und gelangen von dort in extrazelluläres Gewebe des ganzen Körpers. Mittels einer speziellen Affinität der Bandscheibe zu den aufgespaltenen Inhaltsstoffen, insbesondere hinsichtlich pH-Wert und Ladung, werden die Inhaltsstoffe an der Bandscheibe in besonders hoher Konzentration angereichert. Dort werden die Inhaltsstoffe in der Bandscheibe schnell eingebaut. Dabei dient Collagen zum Aufbau von Faserringcollagen. Kieselsäure verfestigt und stabilisiert die Verbindungen, insbesondere mittels einer Förderung der Vernetzung von Collagen. Der Faserring wird damit optimal gestärkt. Hyaluronsäure und N-Acetyl-Glukosamin, die teilweise vor einem Einbau in die Bandscheibe und teilweise in der Bandscheibe zu einem kompletten Molekül zusammengesetzt werden, binden extrem viel Wasser. Das Volumen des Gallertkerns wird somit vergrößert, was zu einem zunehmenden Abstand zwischen den Wirbelkörpern führen kann.

Ferner ist die orale Verabreichungsform erfindungsgemäß vorteilhaft dazu angepasst, N-Acetyl-Glukosamin in einer Dosis pro Tag von 100 Milligramm bis 700 Milligramm, bevorzugt von 200 Milligramm bis 500 Milligramm und besonders bevorzugt von 300 Milligramm bereitzustellen. Es hat sich erfindungsgemäß gezeigt, dass N-Acetyl-Glukosamin in einer solchen Dosierung die beste Wirkung erreicht. Ein Erhöhen der Dosis bringt keine nennenswerte Steigerung der Wirkung. Verglichen mit dem als Nahrungsergänzungsmittel bekannten Glukosaminsulfat ist die erfindungsgemäße Dosis nur ein Drittel so hoch.

Darüber hinaus ist die Erfindung auf ein Nahrungsergänzungsmittel mit erfindungsgemäßen Zusammensetzungen gerichtet. Damit können dem Körper die erfindungsgemäßen Zusammensetzungen einfach über einen längeren Zeitraum hinweg zugeführt werden. Ein oftmals bestehender Mangel mit notwendigen Aufbaustoffen kann ausgeglichen werden. Bevorzugt kann vor allem die Bandscheibe in ihrem frühen Verschleiß gestärkt und damit erhalten werden.

Dazu eignen sich die erfindungsgemäßen Zusammensetzungen auch für ein Arzneimittel, ein diätetisches Lebensmittel oder ein Medizinprodukt. Als Arzneimittel haben die erfindungsgemäßen Zusammensetzungen bereits beschriebene therapeutische Zwecke, nämlich die Prophylaxe und/oder Behandlung von Rückenschmerzen, die auf degenerativen Veränderungen der Bandscheiben beruhen, insbesondere von degenerativen Wirbelsäulenbeschwerden. Bevorzugt ist eine Behandlung von lumbaler Osteochondrose.

Als diätetisches Lebensmittel sind die erfindungsgemäßen Zusammensetzungen ein Lebensmittel für besondere medizinische Zwecke, die besonderen Ernährungserfordernissen von Personen dienen, die an bestimmten Krankheiten, Störungen oder Beschwerden leiden. Zudem ist das diätetische Lebensmittel ein Lebensmittel für eine besondere Ernährung, das auf besondere Weise verarbeitet oder formuliert und für eine diätetische Behandlung von Patienten gedacht ist.

Als Medizinprodukt sind die erfindungsgemäßen Zusammensetzungen ein Stoff, der zu medizinisch therapeutischen Zwecken verwendet wird. Dabei ist dessen bestimmungsgemäße Hauptwirkung physikalisch oder physikochemisch.

Entsprechend ist die Erfindung auch auf eine Verwendung der erfindungsgemäßen Zusammensetzungen als Arzneimittel, als diätetisches Lebensmittel oder als Medizinprodukt gerichtet.

### Kurzbeschreibung der Zeichnungen

Nachfolgend wird ein Ausführungsbeispiel der erfindungsgemäßen Lösung anhand der beigefügten schematischen Zeichnung näher erläutert. Es zeigt:
- Fig. 1: eine schematische Übersicht eines Ausführungsbeispiels einer erfindungsgemäßen physiologisch aktiven Zusammensetzung,
- Fig. 2: eine schematische Verfahrensübersicht zum Herstellen des Ausführungsbeispiels gemäß Fig. 1 und
- Fig. 3: eine schematische Verfahrensübersicht zum Herstellen von N-Acetyl-Glukosamin für das Ausführungsbeispiel gemäß Fig. 1.

### Detaillierte Beschreibung der Ausführungsbeispiele

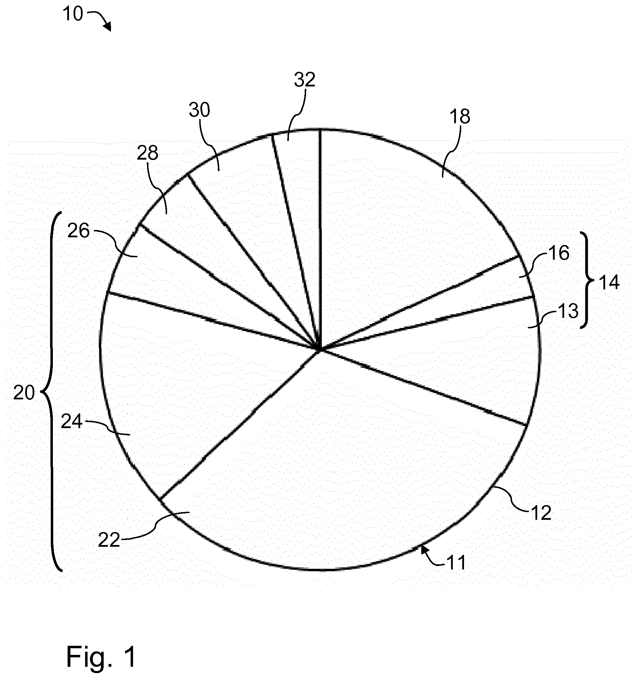

Fig. 1 veranschaulicht eine physiologisch aktive Zusammensetzung 10, wie sie in einer Kapsel 11 als orale Verabreichungsform enthalten ist. Die Kapsel 11 dient als Nahrungsergänzungsmittel für einen Erhalt und Aufbau der Bandscheibe.

Dazu weist die Kapsel 11 eine transparente und harte Kapselhülle 12 aus Hydroxypropylmethylcellulose auf. Im Inneren der Kapselhülle 12 befindet sich die Zusammensetzung 10 als Wirkstoffmischung mehrerer Komponenten.

Eine erste Komponente der Zusammensetzung 10 ist Kieselsäure 13, die in Form von natürlichem Siliziumdioxid mit einem Massenanteil von 75 Gewichtsprozent als Bestandteil eines Bambus-Extrakts 14 enthalten ist. Ferner liegt das Bambus-Extrakt 14 in einer Menge von 70,0 Milligramm in der Kapselhülle vor, woraus sich 52,5 Milligramm an natürlichem Siliziumdioxid als Kieselsäure 13 ergeben. Ein Restanteil 16 des Bambus-Extrakts 14 beläuft sich mit 17,5 Milligramm auf 25 Gewichtsprozent bezogen auf das Bambus-Extrakt 14 und umfasst Calcium, Cholin und Betain.

Eine zweite Komponente der Zusammensetzung 10 ist N-Acetyl-Glukosamin 18 bzw. N-Acetyl-D-Glukosamin in einer Menge von 100,0 Milligramm. Damit liegt in Relation zu N-Acetyl-Glukosamin 18 das Bambus-Extrakt 14 in einem Massenverhältnis von 0,7 vor.

Ferner gehört zur Zusammensetzung 10 Collagenhydrolysat 20 in einer Menge von 300,0 Milligramm. Das Collagenhydrolysat 20 umfasst als weitere Komponente Collagen 22 in einem Massenanteil von 60 bis 70 Gewichtsprozent, vorliegend 60 Gewichtsprozent in einer Menge von 180 Milligramm. Dabei ist Collagen 22 hydrolisiert und liegt bevorzugt als Collagen-Typ II vor.

Zudem umfasst das Collagenhydrolysat 20 als weitere Komponente etwa 30 Gewichtsprozent Mukopolysaccharide bzw. Glykosaminoglykane 24 in einer Menge von etwa 90,0 Milligramm. Ferner sind im Collagenhydrolysat 20 etwa 10 Gewichtsprozent Hyaluronsäure 26 in einer Menge von etwa 30 Milligramm als zusätzliche weitere Komponente der Zusammensetzung 10 vorgesehen.

Insgesamt weist das Collagenhydrolysat 20 ein Massenverhältnis zu N-Acetyl-Glukosamin 18 von 3,0 auf.

Darüber hinaus liegt Lysin 28 in einer stabilisierten Form als L-Lysin Hydrochlorid in einer Menge von 35,0 Milligramm als weitere Komponente der Zusammensetzung 10 vor. Bei der Menge an L-Lysin Hydrochlorid von 35,0 Milligramm ergibt sich eine wirksame Menge an L-Lysin von 28,0 Milligramm. Damit weist Lysin 28 in Bezug auf N-Acetyl-Glukosamin 18 ein Massenverhältnis von 0,28 auf.

Als weitere Komponente umfasst die Zusammensetzung 10 L-(+)-Ascorbinsäure bzw. Ascorbinsäure als Vitamin C 30 in einer Menge von 37,5 Milligramm. Dabei ist ein Stabilitätszuschlag von 25 Gewichtsprozent miteingerechnet, um einen auftretenden Verlust an Vitamin C 30 bei einem längeren Lagern auszugleichen. Ein Massenverhältnis von Vitamin C 30 zu N-Acetyl-Glukosamin 18 liegt je nach Lagerungsverlust etwa zwischen 0,38 und 0,30.

Ferner sind 19,5 Milligramm Magnesiumstearat 32 aus pflanzlicher Herkunft als Hilfsmittel in der Zusammensetzung 10 enthalten.

Mit 100,0 Milligramm N-Acetyl-D-Glukosamin 18 der Zusammensetzung 10 pro Kapsel 11 kann bei einer Einnahme von vier Kapseln 11 pro Tag eine besonders wirksame Dosis erreicht werden.

Für eine besonders gute Wirkung, insbesondere bei Akutbeschwerden, sollten die Kapseln 11 dazu in einer Menge von zwei mal zwei Kapseln 11 pro Tag über einen Zeitraum von zwei bis vier Wochen eingenommen werden. Damit wird ein Anfluten der Komponenten als Wirkstoffe bzw. Nährstoffe im Körper erzielt. Danach kann die Einnahme reduziert werden auf zwei Kapseln 11 pro Tag, aufgeteilt auf zwei mal eine Kapsel 11, für eine Dauer von acht Wochen. Bevorzugt ist eine Einnahmedauer von zwölf Wochen. Die Einnahme sollte nach dem Essen mit viel Flüssigkeit erfolgen.

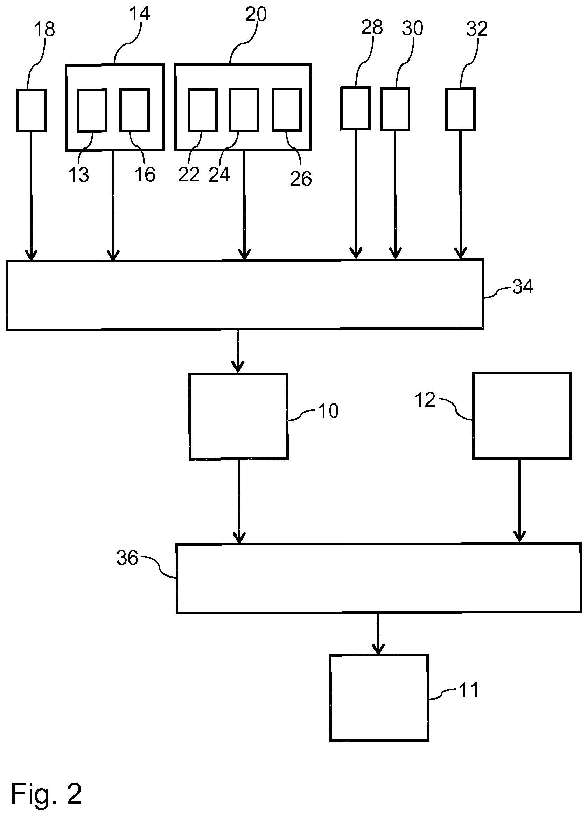

Fig. 2 zeigt ein Herstellungsverfahren für die Kapsel 11. Dabei werden das Bambus-Extrakt 14, N-Acetyl-Glukosamin 18, Collagenhydrolysat 20, Lysin 28 und Vitamin C 30 als Wirkstoff-Komponenten und Magnesiumstearat 32 als Hilfsmittel in einem Schritt 34 bei einer Umgebungstemperatur von etwa 20 °C vermischt. Danach erfolgt in einem Schritt 36 ein Einkapseln der derart erhaltenen vermischten Zusammensetzung 10 in eine leere Kapselhülle 12 bei 20 °C. Es wird die Kapsel 11 erhalten.

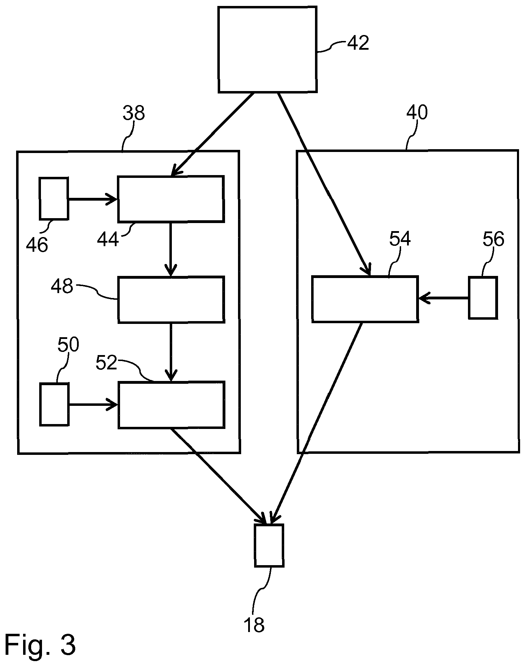

Fig. 3 zeigt zwei grundsätzliche verschiedene Verfahren 38 und 40 zum Herstellen des Wirkstoffs N-Acetyl-D-Glukosamin 18. In beiden Fällen dient natürliches Chitin 42 als Ausgangsstoff.

Chitin 42 ist ein Polysaccharid, das aus durchgängig β-1,4-glykosidisch verknüpften N-Acetyl-Glukosamin-Molekülen besteht. Damit ist Chitin 42 zum Gewinnen von N-Acetyl-Glukosamin 18 besonders gut geeignet. Es wird in beiden Verfahren 38 und 40 Chitin 42 verwendet, das aus Schalen von Krebstieren stammt.

Das eine Verfahren 38 ist ein teilsynthetisches Verfahren, in dem natürliches Chitin 42 einem Schritt 44 einer sauren Hydrolyse bzw. Spaltung unterworfen wird. Dabei wird Chitin 42 mittels einer starken Säure 46 in seine Einzelbausteine gespalten. Die Säure 46 bewirkt zusätzlich eine Abspaltung der Acetyl-Gruppe vom Stickstoff des N-Acetyl-Glukosamins 18.

In einer ersten Variante für vorliegendes Ausführungsbeispiel wird als Säure 46 konzentrierte Salzsäure verwendet. Damit entsteht bei dem Schritt 44 der sauren Hydrolyse Glukosaminhydrochlorid als Zwischenprodukt 48, das nachfolgend konzentriert, kristallisiert und gereinigt wird. Das derart gewonnene Zwischenprodukt 48 Glukosaminhydrochlorid wird in Wasser gelöst und mit Acetanhydrid bzw. Essigsäureanhydrid 50 in einem Schritt 52 einer Re-Acetylierung zu N-Acetyl-Glukosamin 18 umgesetzt. Nach einer Kristallisation und Reinigung mittels Ethanol wird N-Acetyl-Glukosamin 18 in einer Reinheit von 97,5 Prozent erhalten.

In einer alternativen Variante wird in dem Verfahren 38 Schwefelsäure statt Salzsäure als starke Säure 46 verwendet, was zu Glukosaminsulfat als Zwischenprodukt 48 führt.

In einer weiteren alternativen Variante wird N-Acetyl-Glukosamin 18 mittels des anderen Verfahrens 40 direkt aus einer Spaltung des natürlichen Chitins 42 gewonnen. Dazu wird Chitin 42 mit Phosphorsäure gequollen, anschließend neutralisiert und in einem Schritt 54 einer enzymatischen Hydrolyse mittels eines Enzyms 56 unterworfen. Das Enzym 56 ist Chitinase. Nach Abschluss der Hydrolyse und nachfolgender Filtration und Kristallisation steht N-Acetyl-Glukosamin 18 mit einem vollkommen natürlichen Ursprung besonders rein, schnell und einfach zur Verfügung.

Abschließend sei angemerkt, dass sämtlichen Merkmalen, die in den Anmeldungsunterlagen und insbesondere in den abhängigen Ansprüchen genannt sind, trotz des vorgenommenen formalen Rückbezugs auf einen oder mehrere bestimmte Ansprüche, auch einzeln oder in beliebiger Kombination eigenständiger Schutz zukommen soll.

### Bezugszeichenliste

- 10: Zusammensetzung
- 11: Kapsel
- 12: Kapselhülle
- 13: Kieselsäure
- 14: Bambus-Extrakt
- 16: Restanteil Bambus-Extrakt
- 18: N-Acetyl-Glukosamin
- 20: Collagenhydrolysat
- 22: Collagen
- 24: Mukopolysaccharide bzw. Glykosaminoglykane
- 26: Hyaluronsäure
- 28: Lysin
- 30: Vitamin C
- 32: Magnesiumstearat
- 34: Schritt des Vermischens
- 36: Schritt des Einkapseln
- 38: Verfahren zum Herstellen von N-Acetyl-Glukosamin
- 40: Verfahren zum Herstellen von N-Acetyl-Glukosamin
- 42: Chitin
- 44: Schritt einer sauren Hydrolyse
- 46: starke Säure
- 48: Zwischenprodukt
- 50: Acetanhydrid
- 52: Schritt einer Re-Acetylierung
- 54: Schritt einer enzymatischen Hydrolyse
- 56: Enzym

## Patentansprüche

1. N-Acetyl-Glukosamin umfassende physiologisch aktive Zusammensetzung zur Anwendung in einem Verfahren zur Prophylaxe und/oder Behandlung von Rückenschmerzen, die auf degenerativen Veränderungen der Bandscheiben beruhen,
wobei die Zusammensetzung zusätzlich Hyaluronsäure umfasst, welche als Bestandteil eines Collagenhydrolysats vorgesehen ist.

2. Zusammensetzung zur Anwendung nach Anspruch 1,
die zusätzlich Kieselsäure umfasst, welche insbesondere als Bestandteil eines Bambus-Extrakts vorgesehen ist.

3. Zusammensetzung zur Anwendung nach Anspruch 1 oder 2,
die zusätzlich Collagen umfasst, welches insbesondere als Bestandteil des Collagenhydrolysats vorgesehen ist.

4. Zusammensetzung zur Anwendung nach einem der Ansprüche 1 bis 3,
die zusätzlich Lysin und/oder Vitamin C umfasst.

5. Physiologisch aktive Zusammensetzung ,
die mindestens zwei Komponenten umfasst, von denen eine erste Komponente Kieselsäure ist und eine zweite Komponente N-Acetyl-Glukosamin ,
wobei eine weitere Komponente Hyaluronsäure ist, welche als Bestandteil eines Collagenhydrolysats vorgesehen ist.

6. Zusammensetzung nach Anspruch 5,
wobei Kieselsäure als Bestandteil eines Bambus-Extrakts vorgesehen ist.

7. Zusammensetzung nach Anspruch 5 oder 6,
wobei eine weitere Komponente Collagen und/oder eine weitere Komponente Lysin und/oder eine weitere Komponente Vitamin C ist.

8. Zusammensetzung nach einem der Ansprüche 1 bis 7,
die als orale Verabreichungsform gestaltet ist.

9. Nahrungsergänzungsmittel mit einer Zusammensetzung nach einem der Ansprüche 1 bis 8.

## Claims

1. Physiologically active composition comprising N-acetylglucosamine for use in a method for the prophylaxis and/or treatment of back pain caused by degenerative changes in the intervertebral discs,
wherein the composition additionally comprises hyaluronic acid, which is provided in the form of a constituent of a collagen hydrolysate.

2. Composition for use according to claim 1,
which additionally comprises silica, which in particular is provided in the form of a constituent of a bamboo extract.

3. Composition for use according to claim 1 or 2,
which additionally comprises collagen, which is provided in particular in the form of a constituent of the collagen hydrolysate.

4. Composition for use according to any one of claims 1 to 3,
which additionally comprises lysine and/or vitamin C.

5. Physiologically active composition which comprises at least two components, of which a first component is silica and a second component is N-acetylglucosamine,
wherein a further component is hyaluronic acid, which is provided in the form of a constituent of a collagen hydrolysate.

6. Composition according to claim 5,
wherein silica is provided in the form of a constituent of a bamboo extract.

7. Composition according to claim 5 or 6,
wherein a further component is collagen and/or a further component is lysine and/or a further component is vitamin C.

8. Composition according to any one of claims 1 to 7, which is in the form of an oral dosage form.

9. Nutritional supplement comprising a composition according to any one of claims 1 to 8.

## Revendications

1. Composition physiologique active comprenant de la N-acétyl-glucosamine pour une utilisation dans un procédé de prophylaxie et/ou de traitement de douleurs dorsales ayant pour origine des modifications dégénératives des disques intervertébraux ;
la composition comprenant en outre de l'acide hyaluronique prévu sous la forme d'un composé d'hydrolysat de collagène.

2. Composition à utiliser selon la revendication 1, comprenant en outre de la silice notamment prévue sous la forme d'un composé d'extrait de bambou.

3. Composition à utiliser selon la revendication 1 ou 2, comprenant en outre du collagène notamment prévu sous la forme d'un composé d'hydrolysat de collagène.

4. Composition à utiliser selon l'une quelconque des revendications 1 à 3, comprenant en outre de la lysine et/ou de la vitamine C.

5. Composition physiologique active, qui comporte au moins deux composants parmi lesquels un premier composant est la silice et un deuxième composant est le N-acétyl-glucosamine, un composant supplémentaire étant l'acide hyaluronique prévu sous la forme d'un composé d'hydrolysat de collagène.

6. Composition selon la revendication 5, la silice étant prévue sous la forme d'un composé d'extrait de bambou.

7. Composition selon la revendication 5 ou 6, un composé supplémentaire étant le collagène et/ou un composé supplémentaire étant la lysine et/ou un composé supplémentaire étant la vitamine C.

8. Composition selon l'une quelconque des revendications 1 à 7, qui prend une forme d'administration orale.

9. Complément alimentaire avec une composition selon l'une quelconque des revendications 1 à 8.
